# EUROPEAN PATENT APPLICATION

(11) **EP 3 131 056 A1**
(43) Date of publication of application: **15.02.2017**
(21) Application number: 16779751.3
(22) Date of filing: 06.04.2016
(51) Int. Cl.: G06Q 50/24

(54) **MEDICATION HISTORY MANAGEMENT METHOD, MEDICATION HISTORY MANAGEMENT DEVICE AND MEDICATION HISTORY MANAGEMENT PROGRAM**

(30) Priority: 16.04.2015 JP 2015084144
(71) Applicant: Panasonic Healthcare Holdings Co., Ltd., Tokyo 105-8433 (JP)
(72) Inventor: MINEMURA, Atsushi, Ehime 791-0395 (JP); OZAKI, Nobuhiko, Ehime 791-0395 (JP)
(74) Representative: Grünecker Patent- und Rechtsanwälte PartG mbB
(86) International application number: PCT/JP2016/001930
(87) International publication number: WO 2016/166954

(57) **Abstract**

A medication history management method in which a server apparatus (12) transmits/receives information to/from a medical institution terminal (10) and a patient terminal (11) and manages a medication history includes: generating random-number-value information based on a request for the random-number-value information received from a medical institution terminal (10); storing the random-number-value information in a predetermined storage section and displaying the random-number-value information on the medical institution terminal (10); receiving the random value information inputted from the patient terminal (11) and patient-identification information stored in the patient terminal (11) from the patient terminal (11); and reading, when the random value information stored in the predetermined storage section coincides with the random value information received from the patient terminal (11), the medication history information corresponding to the patient-identification information from the predetermined storage section and displaying the medication history information on the medical institution terminal (10).

## Description

### Technical Field

The present invention relates to a medication history management method, a medication history management apparatus, and a medication history management program for managing a medication history.

### Background Art

Medication history information that indicates a medication history has been known as an example of information on a patient handled by medical institutions (e.g., hospitals and pharmacies). This medication history information has been generally recorded in a prescription notebook, which is a paper medium issued and handed to a patient by a pharmacy, but an electronic version of the prescription notebook in which medication history information is converted into electronic data and recorded is also currently spreading widely (e.g., see Patent Literature (hereinafter referred to as "PTL" 1). For example, a pharmacist looks at the electronic version of prescription notebook displayed on a personal computer or the like and checks whether there is a problem with an intake combination of medicines of a patient or checks whether a prescribed medicine can be changed to a generic one. Alternatively, for example, a medical doctor looks at the electronic version of prescription notebook displayed on a personal computer or the like and checks medicines taken by a patient in the past or how contents of a previously issued prescription have been changed.

The electronic version of prescription notebook can be browsed not only by medical personnel but also by patients. For example, a patient may browse his/her electronic version of prescription notebook on his/her smartphone or the like using a dedicated application for the electronic version of prescription notebook (hereinafter referred to as "dedicated application"). Note that, the dedicated application is to be installed and operated by smartphone users on their own, such an application needs to be readily available even for users who are unfamiliar with IT technologies.

### Citation List

### Patent Literature

PTL 1
Japanese Patent Application Laid-Open No. 2015-43239

### Summary of Invention

### Technical Problem

Since the electronic version of prescription notebook involves a high degree of privacy, browsing of the prescription notebook may possibly require user authentication. For example, medical personnel perform registration operation, first using a personal computer or the like in advance, and acquire login information (e.g., ID or password) necessary for user authentication. Next, when a patient using the electronic version of prescription notebook with a dedicated application for his/her smartphone visits a hospital, the medical personnel ask information to allow the electronic version of prescription notebook to be browsed from the patient, log in from a personal computer or the like and input the information acquired from the patient. The electronic version of prescription notebook associated with the inputted information is thereby displayed on a screen, allowing the medical personnel to browse the prescription notebook. However, the system mentioned above involves following problems (1) to (3).
(1) Medical personnel need to perform registration operation in advance, need to perform login operation every time they browse the electronic version of prescription notebook, which takes time and effort.
(2) Medical personnel need to ask information to enable the electronic version of prescription notebook to be browsed from the patient every time they browse the electronic version of prescription notebook, need to input the information acquired, which takes time and effort. On the other hand, the patient needs to remember his/her information to be conveyed to the medical personnel, which takes time and trouble. When the patient does not remember his/her information, the patient needs to operate his/her smartphone to display the information to be conveyed to the medical personnel on the smartphone, which takes time and effort.
(3) There may be a concern that a patient might convey information for enabling the patient's electronic version of prescription notebook to be browsed to a person unqualified for browsing (e.g., malicious third party pretending to be medical personnel, or even medical personnel yet unqualified for browsing), whereby the electronic version of prescription notebook may be browsed without consent of the patient at any time, thus posing a security problem.

From the above, it is desirable to save time and effort of the medical personnel and the patient and enable the electronic version of prescription notebook to be browsed without the need for the patient to convey his/her identification information to the medical personnel.

An object of the present invention is to provide a medication history management method, a medication history management apparatus and a medication history management program that allow information to be browsed while saving time and effort of a user and ensuring a high level of security.

### Solution to Problem

A medication history management method according to the present invention is a method in which a server apparatus transmits and receives information to and from a first terminal and a second terminal and manages a medication history, the method including: generating identification-numerical-value information based on a request for the identification-numerical-value information received from the first terminal; storing the identification-numerical-value information in a predetermined storage section and displaying the identification-numerical-value information on the first terminal; receiving the identification-numerical-value information inputted from the second terminal and patient-identification information stored in the second terminal from the second terminal; and reading, when the identification-numerical-value information received from the second terminal is stored in the predetermined storage section, medication history information corresponding to the patient-identification information from the predetermined storage section based on a request for the medication history information received from the first terminal and displaying the medication history information on the first terminal.

A medication history management method according to the present invention is a method in which a first server apparatus communicable with a first terminal and a second terminal, and a second server apparatus communicable with the first terminal manage a medication history, the method including: generating, by the first server apparatus, identification-numerical-value information based on a request for the identification-numerical-value information received from the first terminal; storing, by the first server apparatus, the identification-numerical-value information in a predetermined storage section and displaying, by the first server apparatus, the identification-numerical-value information on the first terminal; receiving, by the first server apparatus, identification-numerical-value information inputted to the second terminal, and patient-identification information and company-identification information stored in the second terminal from the second terminal; identifying, by the first server apparatus, when the identification-numerical-value information received from the second terminal is stored in the predetermined storage section, redirect destination information that enables access to the second server apparatus based on the company-identification information received from the second terminal; generating redirect setting information including the patient-identification information received from the second terminal and the redirect destination information and transmitting the redirect setting information to the first terminal; and reading, by the second server apparatus, when access based on the redirect setting information is made from the first terminal, medication history information corresponding to the patient-identification information included in the redirect setting information from a predetermined storage section and displaying the medication history information on the first terminal.

A medication history management method according to the present invention is a method in which a first server apparatus communicable with a first terminal and a second terminal, and a second server apparatus communicable with the first terminal and the first server apparatus manage a medication history, the method including: generating, by the first server apparatus, identification-numerical-value information based on a request for the identification-numerical-value information received from the first terminal; storing, by the first server apparatus, the identification-numerical-value information in a predetermined storage section and displaying, by the first server apparatus, the identification-numerical-value information on the first terminal; receiving, by the first server apparatus, identification-numerical-value information inputted to the second terminal, and patient-identification information and company-identification information stored in the second terminal from the second terminal; transmitting, by the first server apparatus, when the identification-numerical-value information received from the second terminal is stored in the predetermined storage section and access inquiring about whether or not the company-identification information is received has been received in advance from the second server apparatus, the patient-identification information received from the second terminal to the second server apparatus; transmitting, by the second server apparatus, when the patient-identification information is received from the first server apparatus, redirect destination information that enables access to the second server apparatus to the first server apparatus; generating, by the first server apparatus, redirect setting information including the patient-identification information received from the second terminal and the redirect destination information received from the second server apparatus, and transmitting the redirect setting information to the first terminal; and reading, by the second server apparatus, when access based on the redirect setting information is received from the first terminal, medication history information corresponding to patient-identification information included in the redirect setting information from a predetermined storage section, and displaying by the second server apparatus the medication history information on the first terminal.

A medication history management apparatus according to the present invention is an apparatus that transmits and receives information to and from a first terminal and a second terminal and manages a medication history, the medication history management apparatus including a control section that performs, when a request for identification-numerical-value information is received from the first terminal, control so as to generate the identification-numerical-value information, to store the identification-numerical-value information in a predetermined storage section, and to display the identification-numerical-value information on the first terminal, in which the control section performs control so as to determine, when the identification-numerical-value information inputted from the second terminal and patient-identification information stored in the second terminal are received from the second terminal, whether or not the identification-numerical-value information received from the second terminal is stored in the predetermined storage section, and to read, when the identification-numerical-value information received from the second terminal is stored in the predetermined storage section, medication history information corresponding to the patient-identification information from the predetermined storage section based on the request for the medication history information received from the first terminal and to display the medication history information on the first terminal.

A medication history management apparatus according to the present invention is an apparatus that transmits and receives information to and from a first terminal communicable with a server apparatus configured to manage predetermined medication history information, and a second terminal, and that manages predetermined medication history information, the medication history management apparatus including a control section that performs, when a request for identification-numerical-value information is received from the first terminal, control so as to generate the identification-numerical-value information, to store the identification-numerical-value information in a predetermined storage section, and to display the identification-numerical-value information on the first terminal, in which the control section performs control so as to determine, when the identification-numerical-value information inputted from the second terminal and patient-identification information and company-identification information stored in the second terminal are received from the second terminal, whether or not the identification-numerical-value information received from the second terminal is stored in the predetermined storage section, to identify, when the identification-numerical-value information received from the second terminal is stored in the predetermined storage section, redirect destination information that enables access to the server apparatus based on the company-identification information received from the second terminal, and to generate redirect setting information including the patient-identification information received from the second terminal and the redirect destination information, and to transmit the redirect setting information to the first terminal, in which the redirect setting information is information for the first terminal to request the server apparatus to display medication history information managed by the server apparatus on the first terminal.

A medication history management apparatus according to the present invention is an apparatus that transmits and receives information to and from a first terminal communicable with a server apparatus configured to manage predetermined medication history information, a second terminal and the server apparatus, and that manages predetermined medication history information, the medication history management apparatus including a control section that performs, when a request for identification-numerical-value information is received from the first terminal, control so as to generate the identification-numerical-value information, to store the identification-numerical-value information in a predetermined storage section, and to display the identification-numerical-value information on the first terminal, in which the control section performs control so as to determine, when the identification-numerical-value information inputted from the second terminal, and patient-identification information and company-identification information stored in the second terminal are received from the second terminal, whether or not the identification-numerical-value information received from the second terminal is stored in the predetermined storage section, to transmit, when the identification-numerical-value information received from the second terminal is stored in the predetermined storage section and access inquiring about whether or not the company-identification information is received in advance from the server apparatus, the patient-identification information received from the second terminal to the server apparatus, and to generate, when redirect destination information that enables access to the server apparatus is received from the server apparatus that has received the patient-identification information, redirect setting information including the patient-identification information received from the second terminal and the redirect destination information, and to transmit the redirect setting information to the first terminal, in which the redirect setting information is information for the first terminal to request the server apparatus to display medication history information managed by the server apparatus on the first terminal.

A medication history management program according to the present invention is a program to be executed by a computer that transmits and receives information to and from a first terminal and a second terminal and that manages a medication history, the program causing the computer to execute processing including: generating identification-numerical-value information based on a request for the identification-numerical-value information received from the first terminal; storing the identification-numerical-value information in a predetermined storage section and displaying the identification-numerical-value information on the first terminal; receiving the identification-numerical-value information inputted from the second terminal and patient-identification information stored in the second terminal from the second terminal; and reading, when the identification-numerical-value information received from the second terminal is stored in the predetermined storage section, medication history information corresponding to the patient-identification information from the predetermined storage section based on a request for the medication history information received from the first terminal and displaying the medication history information on the first terminal.

A medication history management program according to the present invention is a program to be executed by a computer that transmits and receives information to and from a first terminal communicable with a server apparatus configured to manage predetermined medication history information, and a second terminal, and that manages predetermined medication history information, the program causing the computer to execute processing including: generating the identification-numerical-value information based on a request for the identification-numerical-value information received from the first terminal; storing the identification-numerical-value information in a predetermined storage section and displaying the identification-numerical-value information on the first terminal; determining, when the identification-numerical-value information inputted from the second terminal, and patient-identification information and company-identification information stored in the second terminal are received from the second terminal, whether or not the identification-numerical-value information received from the second terminal is stored in the predetermined storage section; identifying, when the identification-numerical-value information received from the second terminal is stored in the predetermined storage section, redirect destination information that enables access to the server apparatus based on the company-identification information received from the second terminal; generating redirect setting information including the patient-identification information received from the second terminal and the redirect destination information; and transmitting the redirect setting information to the first terminal, in which the redirect setting information is information for the first terminal to request the server apparatus to display medication history information managed by the server apparatus on the first terminal.

A medication history management program according to the present invention is a program to be executed by a computer that transmits and receives information to and from a first terminal communicable with a server apparatus configured to manage predetermined medication history information, a second terminal, and the server apparatus, and that manages predetermined medication history information, the program causing the computer to execute processing including: generating identification-numerical-value information based on a request for the identification-numerical-value information received from the first terminal; storing the identification-numerical-value information in a predetermined storage section and displaying the identification-numerical-value information on the first terminal; determining, when the identification-numerical-value information inputted from the second terminal, and patient-identification information and company-identification information stored in the second terminal are received from the second terminal, whether or not the identification-numerical-value information received from the second terminal is stored in the predetermined storage section; transmitting, when the identification-numerical-value information received from the second terminal is stored in the predetermined storage section and access inquiring about whether or not the company-identification information is received has been received in advance from the server apparatus, the patient-identification information received from the second terminal to the server apparatus; generating, when redirect destination information that enables access to the server apparatus is received from the server apparatus that has received the patient-identification information, redirect setting information including the patient-identification information received from the second terminal and the redirect destination information; and transmitting the redirect setting information to the first terminal, in which the redirect setting information is information for the first terminal to request the server apparatus to display medication history information managed by the server apparatus on the first terminal.

### Advantageous Effects of Invention

According to the present invention, it is possible to browse information while saving time and effort of a user and ensuring a high level of security.

### Brief Description of Drawings

FIG. 1 is a diagram illustrating an example of a configuration of a medication history management system according to an embodiment of the present invention;
FIG. 2 is a diagram illustrating an example of a configuration of a medical institution terminal according to the embodiment of the present invention;
FIG. 3 is a diagram illustrating an example of a configuration of a patient terminal according to the embodiment of the present invention;
FIG. 4 is a diagram illustrating an example of a configuration of a server apparatus according to the embodiment of the present invention;
FIG. 5 is a diagram illustrating an example of a random-number-value management table according to the embodiment of the present invention;
FIG. 6 is a diagram illustrating an example of a medication-history management table according to the embodiment of the present invention;
FIG. 7 is a diagram illustrating an operation example of a medication history management system according to the embodiment of the present invention;
FIG. 8 is a diagram illustrating an operation example of the medication history management system according to the embodiment of the present invention;
FIG. 9 is a diagram illustrating an operation example of a medication history management system according to a modification of the embodiment of the present invention; and
FIG. 10 is a diagram illustrating an operation example of a medication history management system according to a modification of the embodiment of the present invention.

### Description of Embodiment

Hereinafter, an embodiment of the present invention will be described in detail with reference to the accompanying drawings. Note that the embodiment to be described below is only exemplary, and the present invention is not limited to the embodiment.

### <Configuration of Medication History Management System>

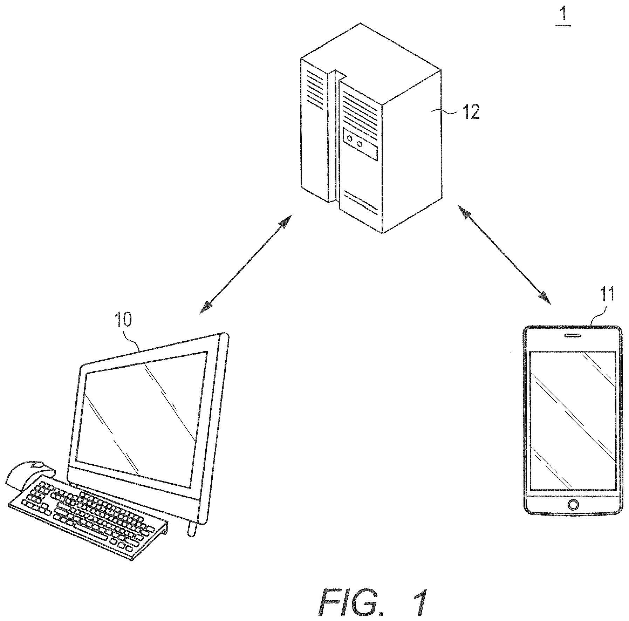

A configuration of medication history management system 1 according to an embodiment of the present invention will be described using FIG. 1. FIG. 1 is a diagram illustrating an example of medication history management system 1 according to the present embodiment.

Medication history management system 1 includes medical institution terminal 10, patient terminal 11 and server apparatus 12.

Medical institution terminal 10 (an example of a first terminal) is an apparatus (e.g., personal computer, smartphone or tablet) installed at a medical institution such as hospital or pharmacy, and used by medical personnel.

Patient terminal 11 (an example of a second terminal) is an apparatus (e.g., personal computer, smartphone or tablet) used by a patient using a medical institution.

Server apparatus 12 (an example of medication history management apparatus) is an apparatus operated and managed by, for example, a provider of services for an electronic version of prescription notebook or a provider of a dedicated application. The dedicated application is application software provided with a browsing function or the like of an electronic version of prescription notebook (medication history information) operating on patient terminal 11.

Server apparatus 12 communicates with medical institution terminal 10 via a predetermined network. Server apparatus 12 communicates with patient terminal 11 via a predetermined network. The predetermined network may be any one of a wired network, a wireless network and a mixture of a wired network and a wireless network.

### <Configuration of Medical Institution Terminal>

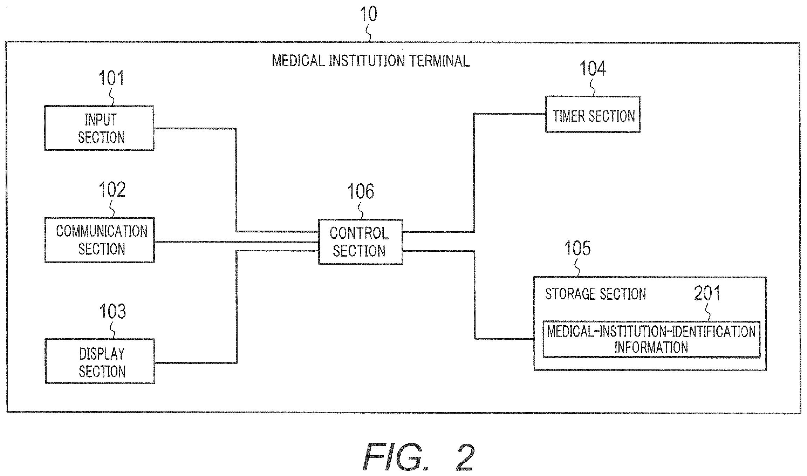

Next, a configuration of medical institution terminal 10 according to an embodiment of the present invention will be described using FIG. 2. FIG. 2 is a diagram illustrating an example of a configuration of medical institution terminal 10 according to the present embodiment.

Medical institution terminal 10 includes input section 101, communication section 102, display section 103, timer section 104, storage section 105 and control section 106.

Input section 101 is an input apparatus such as a touch panel, a keyboard or a mouse. Input section 101 receives an operation by medical personnel and outputs contents of the received operation to control section 106.

Input section 101 receives, for example, an operation of requesting random-number-value information or an operation of requesting medication history information from server apparatus 12. Details of the random-number-value information and medication history information will be described later.

Communication section 102 is a communication apparatus including a transmission circuit, a reception circuit or the like. Communication section 102 communicates with server apparatus 12 via a predetermined network under the control of control section 106.

Communication section 102 transmits, for example, a request for random-number-value information to server apparatus 12. Communication section 102 also transmits, for example, a request for medication history information to server apparatus 12.

Communication section 102 receives, for example, random-number-value information from server apparatus 12. Communication section 102 also receives, for example, medication history information, first processing-result information or second processing-result information from server apparatus 12. Details of the first processing-result information and the second processing-result information will be described later

Display section 103 is a display apparatus such as a display. Display section 103 displays information under the control of control section 106.

Display section 103 displays, for example, a website to access server apparatus 12, random-number-value information, medication history information or the like received by communication section 102 from server apparatus 12.

Timer section 104 is a timer apparatus that measures a predetermined time.

Storage section 105 is a storage device such as a memory or a hard disk apparatus, and stores various kinds of information.

Storage section 105 stores, for example, medical-institution-identification information 201. Medical-institution-identification information 201 is information that can identify a predetermined medical institution.

Storage section 105 stores, for example, information received by communication section 102 from server apparatus 12 (e.g., random-number-value information, medication history information, first processing-result information, or second processing-result information).

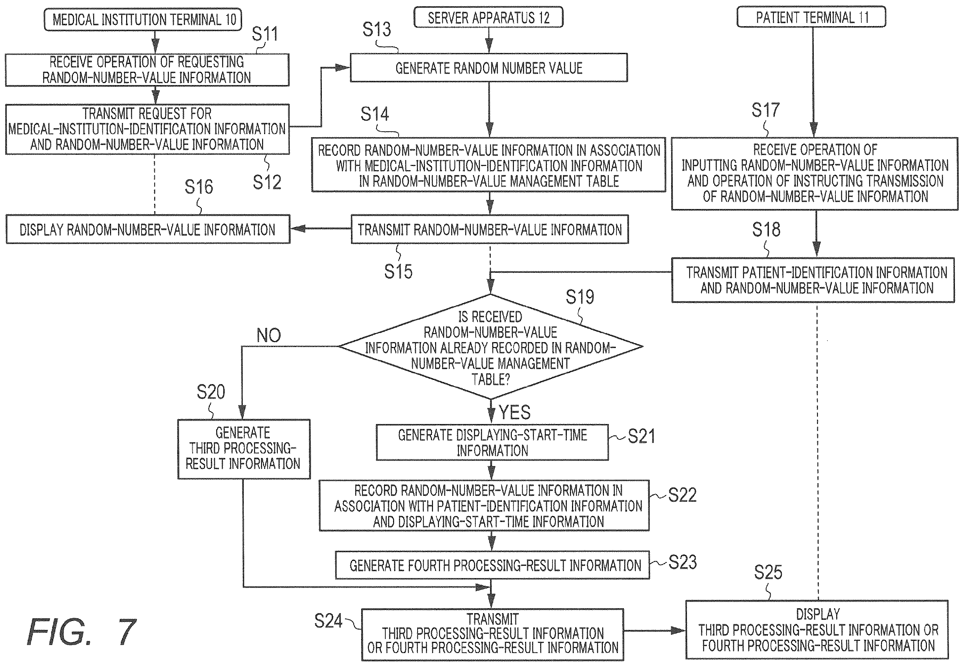

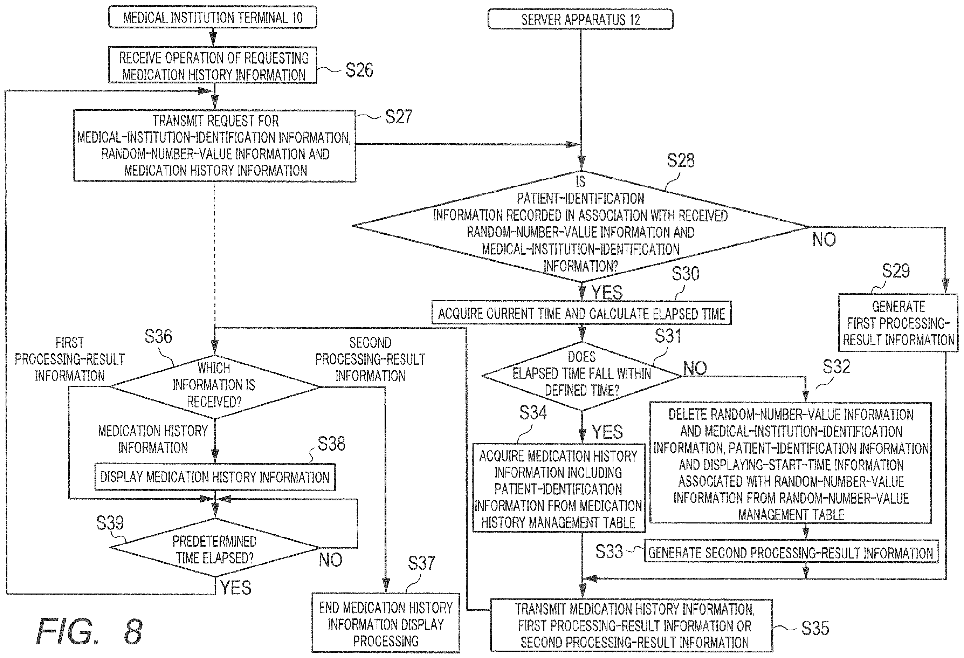

Control section 106 is a control device such as a processor and controls various processes carried out by medical institution terminal 10. Details of control carried out by control section 106 will be described later using FIG. 7 and FIG. 8.

### <Configuration of Patient Terminal>

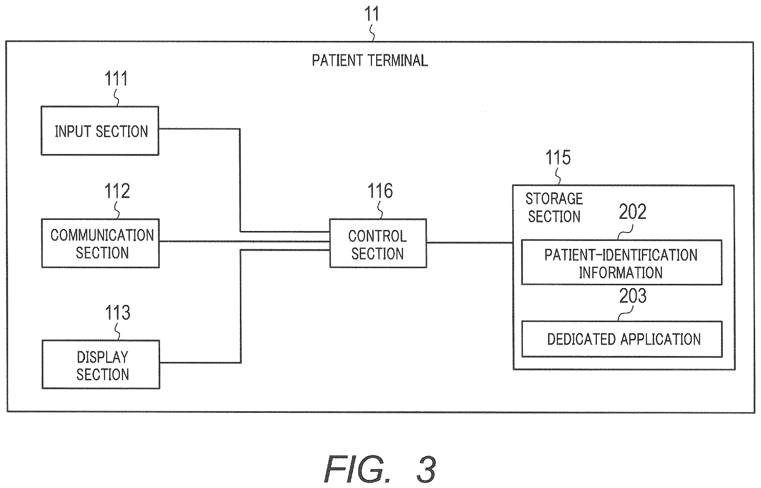

Next, a configuration of patient terminal 11 according to the embodiment of the present invention will be described using FIG. 3. FIG. 3 is a diagram illustrating an example of a configuration of patient terminal 11 according to the present embodiment.

Patient terminal 11 includes input section 111, communication section 112, display section 113, storage section 115 and control section 116.

Input section 111 is an input apparatus such as a touch panel, a keyboard or a mouse. Input section 111 receives an operation by a patient and outputs contents of the received operation to control section 116.

Input section 111 receives, for example, an operation of instructing startup of a dedicated application, an operation of inputting random-number-value information or an operation of instructing transmission of random-number-value information to server apparatus 12.

Communication section 112 is a communication apparatus including an antenna, a transmission circuit, a reception circuit or the like. Communication section 112 performs communication with server apparatus 12 via a predetermined network under the control of control section 116.

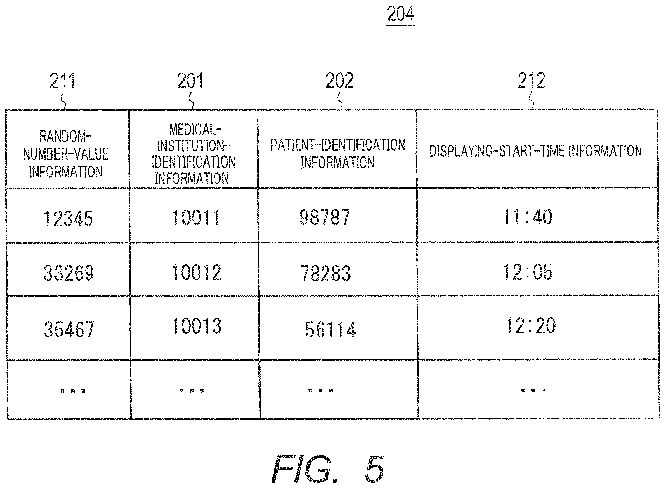

Communication section 112 transmits, for example, patient-identification information 202 (see FIG. 3) and random-number-value information 211 (see FIG. 5) to server apparatus 12. Details of the patient-identification information will be described later.

Communication section 112 receives, for example, third processing-result information and fourth processing-result information from server apparatus 12. Details of the third processing-result information or the fourth processing-result information will be described later.

Display section 113 is a display apparatus such as a display. Display section 113 displays information under the control of control section 116.

Display section 113 displays, for example, a screen for inputting random-number-value information of a dedicated application and processing-result information received by communication section 112 from server apparatus 12.

Storage section 115 is a storage device such as a memory or a hard disk apparatus and stores various kinds of information.

Storage section 115 stores, for example, patient-identification information 202 and dedicated application 203. Patient-identification information 202 is information that can identify a predetermined patient.

Storage section 115 also stores, for example, processing-result information received by communication section 112 from server apparatus 12.

Control section 116 is a control device such as a processor and controls various processes carried out by patient terminal 11. Details of control by control section 116 will be described later using FIG. 7.

### <Configuration of Server Apparatus>

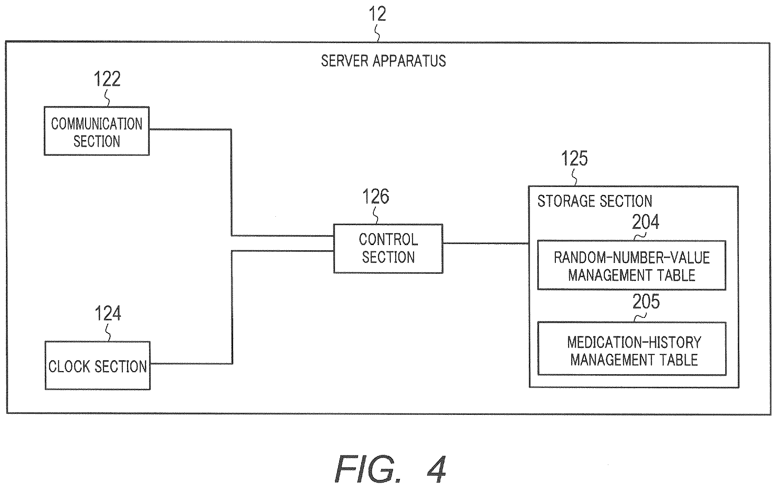

Next, a configuration of server apparatus 12 (an example of a medication history management apparatus) according to the embodiment of the present invention will be described using FIG. 4. FIG. 4 is a diagram illustrating an example of a configuration of server apparatus 12 according to the present embodiment.

Server apparatus 12 includes communication section 122, clock section 124, storage section 125 and control section 126.

Communication section 122 is a communication apparatus including a transmission circuit, a reception circuit or the like. Communication section 122 communicates with medical institution terminal 10 and patient terminal 11 via a predetermined network under the control of control section 126.

Communication section 122 receives, for example, a request for random-number-value information from medical institution terminal 10. Communication section 122 transmits, for example, random-number-value information to medical institution terminal 10.

Communication section 122 also receives, for example, a request for medication history information from medical institution terminal 10. Communication section 122 transmits, for example, one of medication history information, first processing-result information and second processing-result information to medical institution terminal 10.

Communication section 122 also receives, for example, patient-identification information and random-number-value information from patient terminal 11. Communication section 122 also transmits third processing-result information or fourth processing-result information to patient terminal 11.

Clock section 124 is a clock apparatus that counts the current time.

Storage section 125 is a storage device such as a memory or a hard disk apparatus and stores various kinds of information.

Storage section 125 stores information (e.g., random-number-value information, medical-institution-identification information) received by communication section 122 from medical institution terminal 10. Storage section 125 stores, for example, information (e.g., random-number-value information, patient-identification information) received by communication section 122 from patient terminal 11.

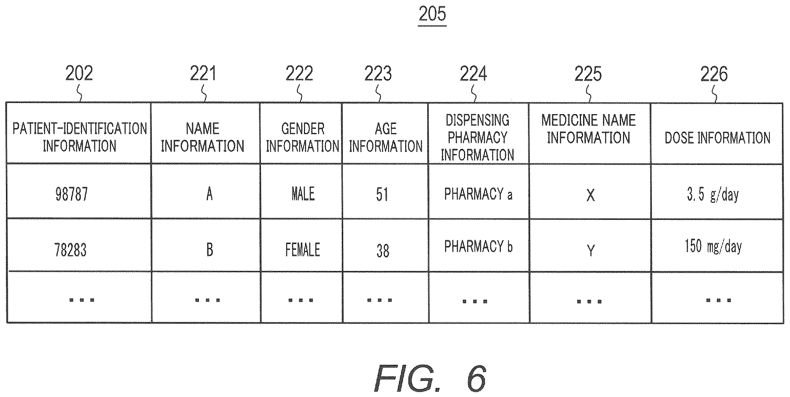

Storage section 125 also stores, for example, random-number-value management table 204 and medication-history management table 205. Medical-institution-identification information 201 is information that can identify a predetermined medical institution. Details of random-number-value management table 204 will be described later using FIG. 5 and details of medication-history management table 205 will be described later using FIG. 6.

Control section 126 is a control device such as a processor and controls various processes carried out by medical institution terminal 10. Details of control by control section 126 will be described later using FIG. 7 and FIG. 8.

### <Random-number-value management table>

Next, random-number-value management table 204 will be described using FIG. 5. FIG. 5 is a diagram illustrating an example of random-number-value management table 204 stored in storage section 125 of server apparatus 12.

As shown in FIG. 5, random-number-value management table 204 associates each item of random-number-value information 211 with medical-institution-identification information 201, patient-identification information 202 and displaying-start-time information 212.

Random-number-value information 211 is information indicating a random number value (an example of identification numerical value) generated by control section 126.

Medical-institution-identification information 201 is identification information indicating a medical institution.

Patient-identification information 202 is identification information indicating a patient.

Displaying-start-time information 212 is information indicating a time at which displaying of medication history information is started, counted by clock section 124.

### <Medication-History Management Table>

Next, medication-history management table 205 will be described using FIG. 6. FIG. 6 is a diagram illustrating an example of medication-history management table 205 stored in storage section 125 of server apparatus 12.

As shown in FIG. 6, medication-history management table 205 associates each item of patient-identification information 202 with medication history information. The medication history information includes name information 221, gender information 222, age information 223, dispensing pharmacy information 224, medicine name information 225 and dose information 226.

Patient-identification information 202 is identification information indicating a patient.

Name information 221 is information indicating the patient's name.

Gender information 222 is information indicating the patient's gender.

Age information 223 is information indicating the patient's age.

Dispensing pharmacy information 224 is information indicating the name of a pharmacy responsible for dispensing.

Medicine name information 225 is information indicating the name of a medicine currently being taken by the patient or the name of a medicine taken by the patient in the past.

Dose information 226 is information indicating the quantity of a medicine being taken by the patient or the quantity of a medicine taken by the patient in the past. Note that the example in FIG. 6 shows a dose per day, but the quantity of a medicine may be a quantity per dosage or a quantity per predetermined period (e.g., two to three days, a week or the like).

Note that the medication history information may include information other than the information shown in FIG. 6. The medication history information may include, for example, information indicating the name of a disease, information indicating the name of a pharmacist, information indicating a comment of the pharmacist, information indicating indications for a medicine, information indicating a usual dose of a medicine and information indicating a method of taking a medicine or the like.

Each item of information included in the medication history information may be updated by an input operation by the medical personnel or the patient or updated by receiving information from an external apparatus (not shown).

### <Operation of Medication History Management System>

Next, an operation of medication history management system 1 according to the embodiment of the present invention will be described using FIG. 7 and FIG. 8. FIG. 7 and FIG. 8 are diagrams illustrating an example of operation of medication history management system 1 (an example of a medication history management method). Medication history management system 1 performs the operation shown in FIG. 7 followed by the operation shown in FIG. 8. A situation in which the operations shown in FIG. 7 and FIG. 8 are performed may be a case where a medical doctor meets a patient and gives a medical examination.

First, the operation example shown in FIG. 7 will be described.

First, input section 101 of medical institution terminal 10 receives an operation of requesting random-number-value information (step S11). For example, the medical personnel access a web page from medical institution terminal 10 to communicate with server apparatus 12 and perform an operation of requesting random-number-value information on the web page. Note that accessing the web page at a dedicated URL itself to communicate with server apparatus 12 may be assumed as an operation of requesting random-number-value information. In that case, the web page is displayed while the random-number-value information is displayed.

Next, control section 106 reads medical-institution-identification information 201 from storage section 105 and controls communication section 102 so as to transmit requests for medical-institution-identification information 201 and random-number-value information to server apparatus 12. Communication section 102 transmits the requests for medical-institution-identification information 201 and random-number-value information to server apparatus 12 (step S12).

Next, when communication section 122 of server apparatus 12 receives the requests for medical-institution-identification information 201 and random-number-value information, control section 126 generates a random number value which is a 5-digit numeric string (step S13). Control section 126 reads random-number-value management table 204 from storage section 125 and confirms whether or not random-number-value information 211 indicating the generated random number value is included in random-number-value management table 204. If the generated random number value is included in random-number-value management table 204, control section 126 generates a random number value again and performs similar confirmation using random-number-value management table 204.

Next, control section 126 associates random-number-value information 211 indicating the generated random number value with medical-institution-identification information 201 received from medical institution terminal 10 and records the associated information in random-number-value management table 204 (step S14). For example, when the generated random number value is "12345" and received medical-institution-identification information 201 is "10011," random-number-value information 211 "12345" is associated with medical-institution-identification information 201 "10011" and recorded as shown in FIG. 5. Note that patient-identification information 202 and displaying-start-time information 212 remain blank (unrecorded) at this point in time.

Next, control section 126 controls communication section 122 so as to transmit random-number-value information 211 to medical institution terminal 10. Communication section 122 transmits random-number-value information 211 to medical institution terminal 10 (step S15).

Next, when communication section 102 of medical institution terminal 10 receives random-number-value information 211, control section 106 causes storage section 105 to store random-number-value information 211 and controls display section 103 so as to display random-number-value information 211. Display section 103 displays random-number-value information 211 (step S16).

Here, the medical personnel, for example, orally inform the patient of random-number-value information 211 displayed on display section 103. The patient starts dedicated application 203 on patient terminal 11 and performs operation of inputting informed random-number-value information 211 and performs operation of instructing transmission of random-number-value information 211 to server apparatus 12. Input section 111 of patient terminal 11 receives those operations (step S17).

Next, control section 116 reads patient-identification information 202 from storage section 115 and controls communication section 112 so as to transmit patient-identification information 202 and random-number-value information 211 to server apparatus 12. Communication section 112 transmits patient-identification information 202 and random-number-value information 211 to server apparatus 12 (step S18).

Next, when communication section 122 of server apparatus 12 receives patient-identification information 202 and random-number-value information 211, control section 126 determines whether or not received random-number-value information 211 is already recorded in random-number-value management table 204 (step S 19).

When the determination result in step S19 shows that random-number-value information 211 is not recorded yet (step S19: NO), control section 126 generates third processing-result information (step S20). The third processing-result information is information including, for example, a message indicating that random-number-value information 211 is not registered yet.

On the other hand, when the determination result in step S19 shows that random-number-value information 211 is already recorded (step S19: YES), control section 126 acquires a current time counted by clock section 124 and generates displaying-start-time information that indicates the current time as the time at which displaying of medication history information is started (hereinafter referred to as "displaying start time") (step S21). The displaying start time acquired here is, for example, a time (an example of a first time) after server apparatus 12 receives random-number-value information 211 and patient-identification information 202 from patient terminal 11.

Next, control section 126 associates random-number-value information 211 received from patient terminal 11 with patient-identification information 202 and displaying-start-time information 212 and records the associated information in random-number-value management table 204 (step S22). For example, when received random-number-value information 211 is "12345," received patient-identification information 202 is "98787" and displaying-start-time information 212 is "11:40," random-number-value information 211 "12345" is associated with patient-identification information 202 "98787" and displaying-start-time information 212 "11:40" and recorded as shown in FIG. 5.

Next, control section 126 generates fourth processing-result information (step S23). The fourth processing-result information is information including a message indicating that random-number-value information 211 is already registered (or may also be a message indicating that a process of displaying medication history information can be started).

Next, control section 126 controls communication section 122 so as to transmit the third processing-result information or fourth processing-result information to patient terminal 11. Communication section 122 transmits third processing-result information or fourth processing-result information to patient terminal 11 (step S24).

Next, when communication section 112 of patient terminal 11 receives the third processing-result information or fourth processing-result information, control section 116 controls display section 113 so as to display the third processing-result information or fourth processing-result information. Display section 113 displays the third processing-result information or fourth processing-result information (step S25).

Here, the patient acts as follows depending on the type of processing-result information displayed on display section 113.

For example, when the third processing-result information indicating a processing failure is displayed, since there is a possibility that wrong random-number-value information 211 may have been inputted, the patient performs an operation of inputting random-number-value information 211 and an operation of instructing transmission of random-number-value information 211 again. Alternatively, since there is also a possibility that the medical personnel may have informed the patient of wrong random-number-value information 211, the patient confirms random-number-value information 211 with the medical personnel and then performs the operation of inputting random-number-value information 211 and the operation of instructing transmission of random-number-value information 211 again.

For example, when the fourth processing-result information indicating a processing success is displayed, the patient, for example, orally informs the medical personnel of the fact that the process has succeeded (e.g., a process of displaying medication history information can start).

Next, an operation example shown in FIG. 8 will be described.

Upon receiving the notice of the processing success from the patient, the medical personnel performs an operation of instructing input section 101 of medical institution terminal 10 to transmit the patient's medication history information. Input section 101 of medical institution terminal 10 receives the operation (step S26).

Next, control section 106 reads medical-institution-identification information 201 and random-number-value information 211 from storage section 105 and controls communication section 102 so as to transmit requests for medical-institution-identification information 201, random-number-value information 211 and the medication history information to server apparatus 12. Communication section 102 transmits requests for medical-institution-identification information 201, random-number-value information 211 and the medication history information to server apparatus 12 (step S27).

Next, when communication section 122 of server apparatus 12 receives the requests for medical-institution-identification information 201, random-number-value information 211 and the medication history information, control section 126 reads random-number-value management table 204 from storage section 125. Control section 126 determines whether or not received random-number-value information 211 and medical-institution-identification information 201 are associated with patient-identification information 202 and recorded in random-number-value management table 204 (step S28).

When the determination result in step S28 shows that patient-identification information 202 is not recorded yet (step S28: NO), control section 126 generates first processing-result information indicating that patient-identification information 202 is not registered yet (step S29).

On the other hand, when the determination result in step S28 shows that patient-identification information 202 is recorded (step S28: YES), control section 126 acquires a current time counted by clock section 124. The current time acquired here is, for example, a time after server apparatus 12 receives the request for medication history information from medical institution terminal 10 (an example of a second time). Control section 126 calculates an elapsed time which is a difference between the time acquired this time and the displaying start time indicated by displaying-start-time information 212 recorded in random-number-value management table 204 (step S30).

Next, control section 126 determines whether or not the calculated elapsed time falls within a defined time (step S31). The defined time is a time during which it is possible to perform a process of displaying medication history information (in other words, the time during which the medical personnel can browse medication history information on medical institution terminal 10), and is set within a range of one to two minutes.

When the determination result in step S31 shows that the elapsed time does not fall within the defined time (step S31: NO), control section 126 deletes received random-number-value information 211, and medical-institution-identification information 201, patient-identification information 202 and displaying-start-time information 212 associated with random-number-value information 211 from random-number-value management table 204 (step S32). Note that when random-number-value information 211 and medical-institution-identification information 201 are recorded but patient-identification information 202 and displaying-start-time information 212 are not recorded in random-number-value management table 204, this corresponds to a case where the patient has not inputted or transmitted random-number-value information 211 which is already recorded in random-number-value management table 204.

Control section 126 generates second processing-result information indicating that the process of displaying medication history information on medical institution terminal 10 will be ended (step S33).

On the other hand, when the determination result in step S31 shows that the elapsed time falls within the defined time (step S31: YES), control section 126 reads medication-history management table 205 from storage section 125 and acquires medication history information including patient-identification information 202 from medication-history management table 205 (step S34). For example, when patient-identification information 202 recorded in random-number-value management table 204 is "98787," name information 221 "A", gender information 222 "male", age information 223 "51", dispensing pharmacy information 224 "pharmacy a", medicine name information 225 "X", and dose information 226 "3.5 g/day" shown in FIG. 6 are acquired as medication history information.

Next, control section 126 controls communication section 122 so as to transmit the medication history information, first processing-result information or second processing-result information to medical institution terminal 10. Communication section 122 transmits the medication history information, first processing-result information or second processing-result information to medical institution terminal 10 (step S35).

Next, when communication section 102 of medical institution terminal 10 receives the medication history information, first processing-result information or second processing-result information, control section 106 determines which information has been received (step S36).

When the determination result in step S36 shows that the first processing-result information has been received (step S36: first processing-result information), a determination process in step S39 is performed. Step S39 will be described later. Note that control section 106 may control display section 103 so as to display first processing-result information before the determination process in step S39. Thus, display section 103 displays, for example, first processing-result information (e.g., a message indicating that patient-identification information 202 is unregistered). The medical personnel who looks at this display can, for example, urge the patient to perform operation of transmitting patient-identification information 202 and random-number-value information 211.

When the determination result in step S36 shows that the second processing-result information has been received (step S36: second processing-result information), control section 106 ends the process of displaying medication history information (step S37). For example, when the second processing-result information is received, if display section 103 displays medication history information, control section 106 controls display section 103 so as to end the display of the medication history information. Furthermore, for example, when the second processing-result information is received, if display section 103 does not display medication history information, control section 106 ends a series of processes. It is thereby possible to limit the display time of the medication history information and automatically hide the medication history information. For this reason, for example, it is possible to prevent the medical personnel who have acquired user identification information from continuing to browse the medication history information without permission of the user.

When the determination result in step S36 shows that the medication history information has been received (step S36: medication history information), control section 106 controls display section 103 so as to display the medication history information and instructs timer section 104 to start measuring a predetermined time. Display section 103 displays the medication history information (step S38). Timer section 104 starts measuring a predetermined time.

Next, control section 106 determines whether or not the predetermined time has elapsed (step S39). For example, by receiving a signal outputted from timer section 104 when a predetermined time has elapsed, control section 106 determines that the predetermined time has elapsed. The predetermined time here is assumed to be a time shorter than the aforementioned defined time.

When the determination result in step S39 shows that the predetermined time has not elapsed (step S39: NO), control section 106 waits.

On the other hand, when the determination result in step S39 shows that the predetermined time has elapsed (step S39: YES), control section 106 controls communication section 102 so as to transmit requests for medical-institution-identification information 201, random-number-value information 211 and the medication history information to server apparatus 12. Communication section 102 transmits requests for medical-institution-identification information 201, random-number-value information 211 and the medication history information to server apparatus 12 again (step S27). It is thereby possible to wait for the patient to transmit patient-identification information 202 and random-number-value information 211. Alternatively, the determination as to whether medical institution terminal 10 should continue or end the process of displaying the medication history information (step S33) can be entrusted to server apparatus 12.

### <Operation and Effects of Embodiment>

As described above, the present embodiment achieves the following operation and effects.

The medical personnel need not perform registration operation before using medication history management system 1 or need not perform login operation every time the medical personnel browses the medication history information, which does not take much time or effort.

The medical personnel need not get information that allows the medication history information to be browsed from the patient and perform the input operation every time the medical personnel browses the medication history information, which does not take much time or effort. Moreover, the patient need not remember the information to be conveyed to the medical personnel, which does not take much time or effort. When the patient does not remember the information, the patient need not perform an operation of causing patient terminal 11 to display the information to be conveyed to the medical personnel, which does not take much time or effort.

When browsing the medication history information, the patient need not convey information to allow medication history information to be browsed to the medical personnel, and it is thereby possible to prevent the patient from conveying the information to a person unqualified for browsing (e.g., malicious third party pretending to be medical personnel, or even medical personnel yet unqualified for browsing). As a result, it is possible to avoid leakage of information and avoid the medication history information from being browsed without consent of the patient at any time, and thereby secure a high level of security. By carrying out an operation of allowing the browsing of the medication history information after visually confirming a viewer who browses the patient's medication history information through a dedicated application, the patient can explicitly express an acknowledgment.

From the above, the present embodiment can save time and effort of the medical personnel and the patient, and allow the browsing of medication history information only after ensuring a high level of security.

### <Modifications of Embodiment>

The embodiment of the present invention has been described so far, but the present invention is not limited to the above embodiment, and various modifications can be made. Hereinafter, such modifications will be described.

### (Modification 1)

In the above-described embodiment, the patient starts dedicated application 203 on patient terminal 11 and performs an operation of inputting random-number-value information 211 and an operation of instructing transmission of random-number-value information 211 using dedicated application 203 (step S17 in FIG. 7), but the present invention is not limited to this. For example, the patient may access a web page to access server apparatus 12 from patient terminal 11 and perform the operation of inputting random-number-value information 211 and the operation of instructing transmission of random-number-value information 211 from the web page.

### (Modification 2)

In the above-described embodiment, the patient performs an operation of inputting random-number-value information 211 and an operation of instructing transmission of random-number-value information 211 (step S17 in FIG. 7), but the present invention is not limited to this. For example, the patient may perform an operation of instructing update of medication history information in addition to the above operation. Through this operation, update information indicating update contents of medication history information inside patient terminal 11 (at least one of name information 221, gender information 222, age information 223, dispensing pharmacy information 224, medicine name information 225 and dose information 226) is transmitted to server apparatus 12 in addition to patient-identification information 202 and random-number-value information 211. Control section 126 of server apparatus 12 updates medication history information recorded in medication-history management table 205 of storage section 125 based on the received update information.

### (Modification 3)

In the above-described embodiment, the medical personnel performs an operation of requesting medication history information (step S26 in FIG. 8) but the present invention is not limited to this. For example, control section 106 of medical institution terminal 10 may perform control so as to cause display section 103 to display random-number-value information 211 and then control communication section 102 so as to transmit requests for medical-institution-identification information 201, random-number-value information 211 and medication history information to server apparatus 12 every time a predetermined time (time estimated to be necessary for the patient to input random-number-value information 211, for example, several tens of seconds to approximately one minute) elapses. This makes it possible to save time and effort for the medical personnel to perform the operation of requesting medication history information. Furthermore, it is possible to save time and effort for the patient to report fourth processing-result information to the medical personnel.

### (Modification 4)

A case has been described in the above-described embodiment where medical-institution-identification information 201 is used, but the present invention is not limited to this. That is, when server apparatus 12 is accessed using a web browser from medical institution terminal 10, the communication connection state is maintained, and consistency in subsequent data exchange between medical institution terminal 10 and server apparatus 12 is maintained, which eliminates the necessity for medical-institution-identification information 201 used as an identifier every time server apparatus 12 is accessed.

### (Modification 5)

In the above-described embodiment, random-number-value management table 204 and medication-history management table 205 are stored in storage section 125 of server apparatus 12, but the present invention is not limited to this. For example, random-number-value management table 204 and medication-history management table 205 may be stored in a storage section of an external apparatus (e.g., database server) installed independently of server apparatus 12. In that case, server apparatus 12 communicates with the external apparatus and reads random-number-value management table 204 and medication-history management table 205.

For example, medication-history management table 205 (medication history information) may be stored in storage section 115 of patient terminal 11. In that case, the medication history information is transmitted from patient terminal 11 to medical institution terminal 10 via server apparatus 12.

### (Modification 6)

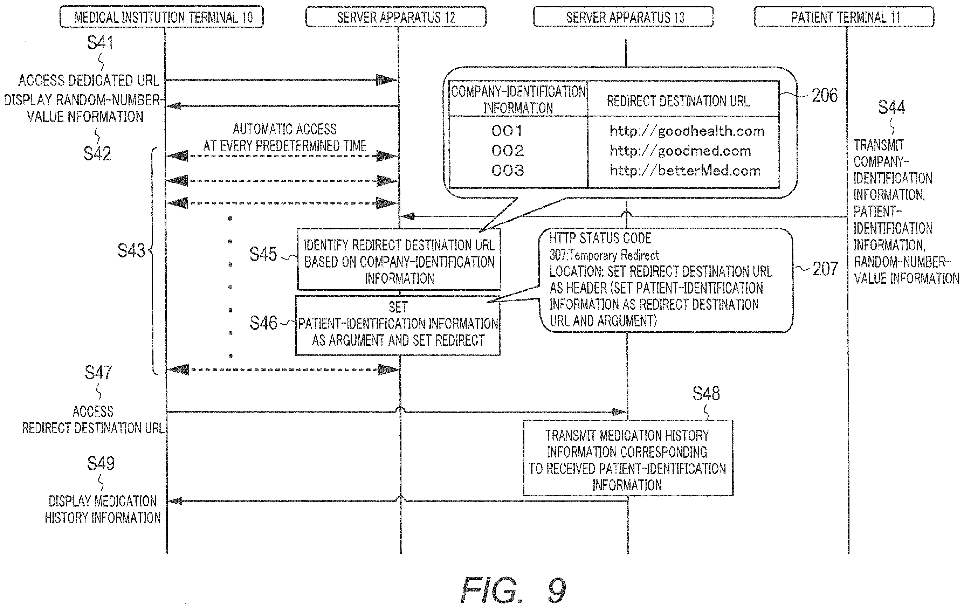

Moreover, the above-described dedicated application may be jointly used by a plurality of service operating companies (hereinafter referred to as "companies") managing medication history information of patients of different pharmacies. This example will be described using FIG. 9.

An example in FIG. 9 includes server apparatus 13 (an example of medication history management apparatus) in addition to medical institution terminal 10, patient terminal 11 and server apparatus 12. An assumption is made that server apparatus 12 is a server apparatus managed by, for example, company A (or managed under a commission from company A) and stores medication history information of patients of pharmacy a. Meanwhile, an assumption is made that server apparatus 13 is a server apparatus managed by company B (or managed under a commission from company B) and stores medication history information of patients of pharmacy b. Note that although FIG. 9 shows only one server apparatus 13, the number of server apparatuses 13 is assumed to be plural.

First, medical institution terminal 10 accesses a dedicated URL based on an operation of the medical personnel (step S41). The dedicated URL refers to an URL that enables access to server apparatus 12.

Next, when access is made from medical institution terminal 10, server apparatus 12 generates and stores random-number-value information 211 and transmits random-number-value information 211 to medical institution terminal 10.

Next, medical institution terminal 10 displays random-number-value information 211 received from server apparatus 12 on display section 103 (step S42). Medical institution terminal 10 conveys displayed random-number-value information 211 to the patient.

Next, medical institution terminal 10 automatically (without involving the operation by the medical personnel) accesses server apparatus 12 at every predetermined time (step S43). Upon receiving the access, server apparatus 12 continues to present the random-number-value information presented above to medical institution terminal 10 unchangingly for a predetermined time, for example, 30 seconds. After a lapse of a certain time, server apparatus 12 presents to medical institution terminal 10 the fact that the random-number-value information has expired. Medical institution terminal 10 automatically accesses server apparatus 12 at every predetermined time so that the medical personnel can be aware of the expiration date of the random-number-value information without performing any operation. Thus, the web page displayed on medical institution terminal 10 is updated as appropriate.

Next, the patient (e.g., a patient of pharmacy b) starts dedicated application 203 (e.g., which is common to companies A and B) from patient terminal 11, inputs random-number-value information 211 conveyed from the medical personnel and performs an operation of instructing server apparatus 12 to transmit random-number-value information 211.

Next, patient terminal 11 reads patient-identification information 202 and company-identification information stored in storage section 115 and transmits the information together with the inputted random-number-value information to server apparatus 12 (step S44). The company-identification information is information that can identify a company (e.g., company A, company B) and is, for example, a character string registered in company-identification information management table 206 which will be described later.

Next, when random-number-value information 211 previously generated and stored coincides with the random-number-value information received from patient terminal 11, server apparatus 12 confirms whether or not server apparatus 12 itself stores (holds) medication history information corresponding to the patient-identification information received from patient terminal 11.

When the above confirmation result shows that server apparatus 12 does not store the medication history information, server apparatus 12 specifies a redirect destination URL based on the company-identification information received from patient terminal 11 (step S45). For example, storage section 105 of server apparatus 12 stores company-identification information management table 206 shown in FIG. 9. Company-identification information management table 206 records company-identification information in association with the redirect destination URL. The redirect destination URL (an example of redirect destination information) refers to a URL that enables access to server apparatus 13, managed by each company.

Next, server apparatus 12 sets patient-identification information 202 received from patient terminal 11 as an argument and sets a redirect (step S46). Server apparatus 12 transmits redirect setting information 207 indicating the set contents (patient-identification information 202 and redirect destination URL) to medical institution terminal 10. The transmission of redirect setting information 207 is performed in response to the access from medical institution terminal 10 in step S43.

Next, medical institution terminal 10 accesses the redirect destination URL indicated in redirect setting information 207 to request server apparatus 13 to display medication history information under its management on medical institution terminal 10 (step S47). Through this access, patient-identification information 202 is transmitted from medical institution terminal 10 to destination server apparatus 13. Note that the redirect setting information can be, in other words, information for requesting server apparatus 13 to display the medication history information under its management on medical institution terminal 10.

Next, server apparatus 13 reads medication history information corresponding to patient-identification information 202 received from medical institution terminal 10, from the storage section and transmits the medication history information to medical institution terminal 10 (step S48).

Next, medical institution terminal 10 displays the medication history information received from server apparatus 13 on display section 103 (step S49). This allows the medical personnel to browse the patient's medication history information. Note that the medication history information may also be displayed within a predetermined number of frames on a website provided by server apparatus 12. In that case, when the aforementioned defined time elapses, only the medication history information within the number of frames remains hidden while the display of the website itself remains unchanged.

Thus, according to the present modification, it is possible to make common the initial access destination from medical institution terminal 10 and supply characteristic medication history information managed by each service operating company compatibly.

### (Modification 7)

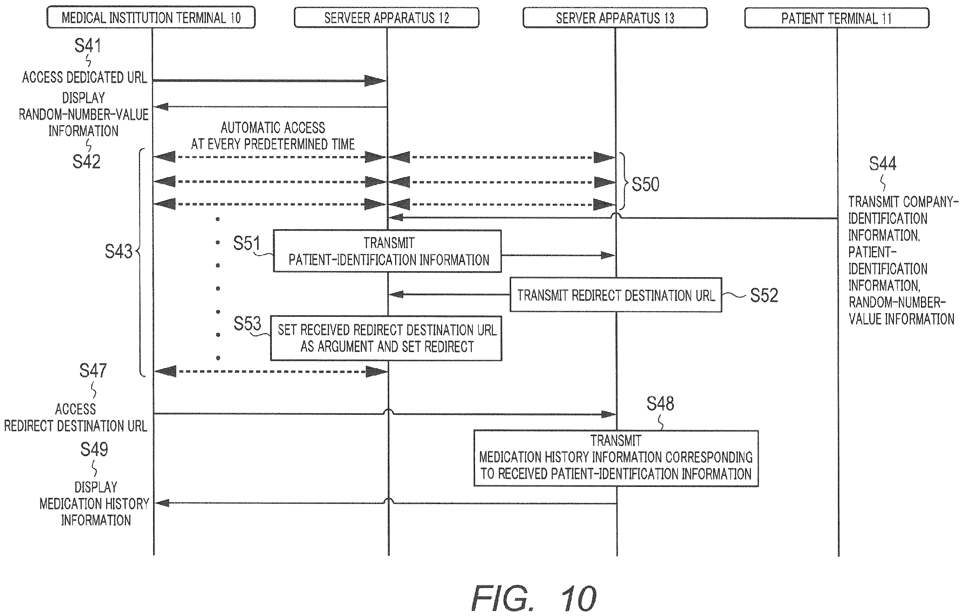

A method for displaying medication history information when a plurality of companies provide their unique dedicated applications respectively will be described using FIG. 10. Note that in FIG. 10, the same processes as those in FIG. 9 are assigned the same reference numerals.

Server apparatus 12 is, for example, a server apparatus managed by company A (or managed under a commission from company A) and is assumed to store medication history information of patients of pharmacy a. On the other hand, an assumption is made that server apparatus 13 is a server apparatus managed by company B (or managed under a commission from company B) and stores medication history information of patients of pharmacy b. Note that although FIG. 10 shows only one server apparatus 13, the number of server apparatuses 13 is assumed to be plural.

Furthermore, company A and company B are assumed to provide their own dedicated applications respectively.

First, medical institution terminal 10 accesses a dedicated URL based on an operation of the medical personnel (step S41). The dedicated URL refers to a URL that enables access to server apparatus 12.

Next, when access is made from medical institution terminal 10, server apparatus 12 generates and stores random-number-value information 211 and transmits random-number-value information 211 to medical institution terminal 10.

Next, medical institution terminal 10 displays random-number-value information 211 received from server apparatus 12 on display section 103 (step S42). Medical institution terminal 10 conveys displayed random-number-value information 211 to the patient.

Next, medical institution terminal 10 automatically (without involving the operation by the medical personnel) accesses server apparatus 12 at every predetermined time (step S43). Upon receiving the access, server apparatus 12 continues to present the random-number-value information presented above to medical institution terminal 10 unchangingly for a predetermined time, for example, 30 seconds. After a lapse of a certain time, server apparatus 12 presents to medical institution terminal 10 the fact that the random-number-value information has expired. Medical institution terminal 10 automatically accesses server apparatus 12 at every predetermined time so that the medical personnel can be aware of the expiration date of the random-number-value information without performing any operation. Thus, the web page displayed on medical institution terminal 10 is updated as appropriate.

Server apparatus 13 automatically accesses server apparatus 12 at every predetermined time (step S50). This access is intended to inquire about, for example, whether or not server apparatus 12 has received company-identification information indicating company B from patient terminal 11.

Next, the patient (e.g., the patient of pharmacy b) starts a dedicated application provided by company B from patient terminal 11, inputs random-number-value information 211 conveyed from the medical personnel and performs an operation of instructing transmission of random-number-value information 211. Note that, for example, when the dedicated application provided by company B is started, patient terminal 11 reads company-identification information (e.g., company-identification information of company B) from the dedicated application and stores the company-identification information in storage section 115.

Next, patient terminal 11 reads patient-identification information 202 and company-identification information from storage section 115 and transmits the information together with the inputted random-number-value information to server apparatus 12 (step S44).

Next, since server apparatus 12 has received the company-identification information of company B, server apparatus 12 recognizes that server apparatus 12 does not store the medication history information corresponding to patient-identification information 202 received together with the company-identification information. Server apparatus 12 confirms whether or not random-number-value information 211 previously generated and stored coincides with the random-number-value information received from patient terminal 11.

When the confirmation result shows that both items of the random-number-value information coincide with each other, server apparatus 12 transmits the patient-identification information received from patient terminal 11 to server apparatus 13 as a response to the inquiry received in step S50 (step S51).

Next, upon receiving patient-identification information from server apparatus 12, server apparatus 13 stores the patient-identification information, prepares a display site page or the like that displays medication history information of the patient and transmits a URL of the display site page as a redirect destination URL to server apparatus 12 (step S52). The redirect destination URL (an example of redirect destination information) referred to here is a URL that enables access to server apparatus 13.

Next, upon receiving the redirect destination URL from server apparatus 13, server apparatus 12 sets the redirect destination URL as an argument and sets a redirect (step S53). Server apparatus 12 transmits redirect setting information 207 indicating the set contents (patient-identification information 202 and redirect destination URL) to medical institution terminal 10. The transmission of redirect setting information 207 is performed in response to the access from medical institution terminal 10 in step S43.

Next, medical institution terminal 10 accesses the redirect destination URL indicated in redirect setting information 207 to request server apparatus 13 to display medication history information under its management on medical institution terminal 10 (step S47). Through this access, patient-identification information 202 is transmitted from medical institution terminal 10 to destination server apparatus 13. Note that the redirect setting information can be, in other words, information for requesting server apparatus 13 to display the medication history information under its management on medical institution terminal 10.

Next, server apparatus 13 reads medication history information corresponding to patient-identification information 202 received from medical institution terminal 10, from the storage section and transmits the medication history information to medical institution terminal 10 (step S48).

Next, medical institution terminal 10 displays the medication history information received from server apparatus 13 on display section 103 (step S49). This allows the medical personnel to browse the patient's medication history information. Note that the medication history information may also be displayed within a predetermined number of frames on a website provided by server apparatus 12. In that case, when the aforementioned defined time elapses, only the medication history information within the number of frames remains hidden while the website itself remains unchanged.

Thus, according to the present modification, it is possible to make common the initial access destination from medical institution terminal 10 and supply characteristic medication history information managed by each service operating company compatibly.

### (Modification 8)

In the above-described embodiment, the medical personnel can browse medication history information for a defined time set in advance, but the defined time may be extended according to an instruction of the medical personnel.

For example, when performing an operation of requesting random-number-value information, the medical personnel inputs the name of a medical doctor on medical institution terminal 10 (step S11 in FIG. 7). This information on the name of the medical doctor together with the requests for medical-institution-identification information and random-number-value information is transmitted to server apparatus 12 (step S12 in FIG. 7). Server apparatus 12 records random-number-value information, medical-institution-identification information, information on the name of the medical doctor in association with each other in random-number-value management table 204 (step S14 in FIG. 7).

After that, server apparatus 12 determines whether or not the random-number-value information and the medical-institution-identification information received from medical institution terminal 10 are recorded in association with the patient-identification information and the information on the name of the medical doctor (step S28 in FIG. 8). When the determination result shows that the patient-identification information and the information on the name of the medical doctor are recorded in association, server apparatus 12 calculates the defined time extended by adding a predetermined time to the aforementioned defined time. For example, when the defined time is one minute, server apparatus 12 adds two minutes corresponding to an extension to the defined time and changes the defined time from one minute to three minutes (e.g., when the patient is receiving a medical examination from the medical doctor). Alternatively, when the defined time is, for example, one minute, server apparatus 12 adds 29 minutes corresponding to an extension to the defined time and changes the defined time from one minute to 30 minutes (a case where the medical doctor checks medication history information of the patient in advance while the patient is waiting in a waiting room or the like before examination).

In step S31 in FIG. 8, server apparatus 12 determines whether or not the elapsed time falls within the extended defined time.

Note that although the present modification has taken a case where the medical personnel inputs the name of the medical doctor as an example, the present invention is not limited to this, and the medical personnel may input, for example, instruction information for extending the defined time.

By this means, in the present modification, the medical personnel inputs predetermined information in advance, and it is thereby possible to extend the time for browsing medication history information.

### (Modification 9)

In the above embodiment, server apparatus 12 may allow the browsing of medication history information for access from predetermined application software or web browser on medical institution terminal 10.

The modifications have been described so far. The above modifications may be combined arbitrarily.

Note that the respective functions of medical institution terminal 10, patient terminal 11, server apparatus 12 and server apparatus 13 in the aforementioned embodiment and modifications can be implemented by a computer program. More specifically, the aforementioned respective functions can be implemented by a central processing unit (CPU) provided for a computer copying a program stored in a storage apparatus to a random access memory (RAM), sequentially reading instructions included in the program from the RAM and executing the instructions.

This disclosure of Japanese Patent Application No. 2015-084144, filed on April 16, 2015, the contents of which including the specification and drawings are incorporated herein by reference in its entirety.

### Industrial Applicability

The medication history management method, the medication history management apparatus and the medication history management program according to the present invention are suitable for use in managing medication histories.

### Reference Signs List

- 1: Medication history management system
- 10: Medical institution terminal
- 11: Patient terminal
- 12, 13: Server apparatus
- 101, 111: Input section
- 102, 112, 122: Communication section
- 103, 113: Display section
- 104: Timer section
- 105, 115, 125: Storage section
- 106, 116, 126: Control section
- 124: Clock section
- 201: Medical-institution-identification information
- 202: Patient-identification information
- 203: Dedicated application
- 204: Random-number-value management table
- 205: Medication-history management table
- 206: Company-identification information management table
- 207: Redirect setting information
- 211: Random-number-value information
- 212: Displaying-start-time information
- 221: Name information
- 222: Gender information
- 223: Age information
- 224: Dispensing pharmacy information
- 225: Medicine name information
- 226: Dose information

## Claims

1. A medication history management method in which a server apparatus transmits and receives information to and from a first terminal and a second terminal and manages a medication history, the method comprising:
generating identification-numerical-value information based on a request for the identification-numerical-value information received from the first terminal;
storing the identification-numerical-value information in a predetermined storage section and displaying the identification-numerical-value information on the first terminal;
receiving the identification-numerical-value information inputted from the second terminal and patient-identification information stored in the second terminal from the second terminal; and
reading, when the identification-numerical-value information received from the second terminal is stored in the predetermined storage section, medication history information corresponding to the patient-identification information from the predetermined storage section based on a request for the medication history information received from the first terminal and displaying the medication history information on the first terminal.

2. The medication history management method according to claim 1, further comprising:
displaying, when the identification-numerical-value information received from the second terminal is not stored in the predetermined storage section, processing-result information indicating that the identification-numerical-value information received from the second terminal is unregistered, on the second terminal; and
storing, when the identification-numerical-value information received from the second terminal is stored in the predetermined storage section, the patient-identification information in association with the identification-numerical-value information stored in the predetermined storage section and displaying processing-result information indicating that the identification-numerical-value information received from the second terminal is already registered, on the second terminal.

3. The medication history management method according to claim 2, further comprising displaying, upon reception of a request for the medication history information from the first terminal, when the patient-identification information is not recorded in association with the identification-numerical-value information stored in the predetermined storage section, processing-result information indicating that the patient-identification information is unregistered, on the first terminal.

4. The medication history management method according to claim 2, further comprising:
calculating, upon reception of a request for the medication history information from the first terminal, when the patient-identification information is recorded in association with the identification-numerical-value information stored in the predetermined storage section, an elapsed time from a first time after reception of the identification-numerical-value information from the second terminal to a second time after reception of a request for the medication history information from the first terminal;
deleting, when the elapsed time exceeds a predetermined time, the identification-numerical-value information stored in the predetermined storage section and the patient-identification information associated with the identification-numerical-value information and displaying, on the first terminal, processing-result information indicating termination of a process of displaying the medication history information on the first terminal; and
reading, when the elapsed time does not exceed a predetermined time, the medication history information corresponding to the patient-identification information from the predetermined storage section and displaying the medication history information on the first terminal.

5. A medication history management method in which a first server apparatus communicable with a first terminal and a second terminal, and a second server apparatus communicable with the first terminal manage a medication history, the method comprising:
generating, by the first server apparatus, identification-numerical-value information based on a request for the identification-numerical-value information received from the first terminal;
storing, by the first server apparatus, the identification-numerical-value information in a predetermined storage section and displaying, by the first server apparatus, the identification-numerical-value information on the first terminal;
receiving, by the first server apparatus, identification-numerical-value information inputted to the second terminal, and patient-identification information and company-identification information stored in the second terminal from the second terminal;
identifying, by the first server apparatus, when the identification-numerical-value information received from the second terminal is stored in the predetermined storage section, redirect destination information that enables access to the second server apparatus based on the company-identification information received from the second terminal;
generating redirect setting information including the patient-identification information received from the second terminal and the redirect destination information and transmitting the redirect setting information to the first terminal; and
reading, by the second server apparatus, when access based on the redirect setting information is made from the first terminal, medication history information corresponding to the patient-identification information included in the redirect setting information from a predetermined storage section and displaying the medication history information on the first terminal.

6. A medication history management method in which a first server apparatus communicable with a first terminal and a second terminal, and a second server apparatus communicable with the first terminal and the first server apparatus manage a medication history, the method comprising:
generating, by the first server apparatus, identification-numerical-value information based on a request for the identification-numerical-value information received from the first terminal;
storing, by the first server apparatus, the identification-numerical-value information in a predetermined storage section and displaying, by the first server apparatus, the identification-numerical-value information on the first terminal;
receiving, by the first server apparatus, identification-numerical-value information inputted to the second terminal, and patient-identification information and company-identification information stored in the second terminal from the second terminal;
transmitting, by the first server apparatus, when the identification-numerical-value information received from the second terminal is stored in the predetermined storage section and access inquiring about whether or not the company-identification information is received has been received in advance from the second server apparatus, the patient-identification information received from the second terminal to the second server apparatus;
transmitting, by the second server apparatus, when the patient-identification information is received from the first server apparatus, redirect destination information that enables access to the second server apparatus to the first server apparatus;
generating, by the first server apparatus, redirect setting information including the patient-identification information received from the second terminal and the redirect destination information received from the second server apparatus, and transmitting the redirect setting information to the first terminal; and
reading, by the second server apparatus, when access based on the redirect setting information is received from the first terminal, medication history information corresponding to patient-identification information included in the redirect setting information from a predetermined storage section, and displaying by the second server apparatus the medication history information on the first terminal.

7. A medication history management apparatus that transmits and receives information to and from a first terminal and a second terminal and manages a medication history,
the medication history management apparatus comprising a control section that performs, when a request for identification-numerical-value information is received from the first terminal, control so as to generate the identification-numerical-value information, to store the identification-numerical-value information in a predetermined storage section, and to display the identification-numerical-value information on the first terminal, wherein:
the control section performs control so as to determine, when the identification-numerical-value information inputted from the second terminal and patient-identification information stored in the second terminal are received from the second terminal, whether or not the identification-numerical-value information received from the second terminal is stored in the predetermined storage section, and to read, when the identification-numerical-value information received from the second terminal is stored in the predetermined storage section, medication history information corresponding to the patient-identification information from the predetermined storage section based on the request for the medication history information received from the first terminal and to display the medication history information on the first terminal.

8. A medication history management apparatus that transmits and receives information to and from a first terminal communicable with a server apparatus configured to manage predetermined medication history information, and a second terminal, and that manages predetermined medication history information,
the medication history management apparatus comprising a control section that performs, when a request for identification-numerical-value information is received from the first terminal, control so as to generate the identification-numerical-value information, to store the identification-numerical-value information in a predetermined storage section, and to display the identification-numerical-value information on the first terminal, wherein
the control section performs control so as to determine, when the identification-numerical-value information inputted from the second terminal and patient-identification information and company-identification information stored in the second terminal are received from the second terminal, whether or not the identification-numerical-value information received from the second terminal is stored in the predetermined storage section,
to identify, when the identification-numerical-value information received from the second terminal is stored in the predetermined storage section, redirect destination information that enables access to the server apparatus based on the company-identification information received from the second terminal, and
to generate redirect setting information including the patient-identification information received from the second terminal and the redirect destination information, and to transmit the redirect setting information to the first terminal, wherein
the redirect setting information is information for the first terminal to request the server apparatus to display medication history information managed by the server apparatus on the first terminal.

9. A medication history management apparatus that transmits and receives information to and from a first terminal communicable with a server apparatus configured to manage predetermined medication history information, a second terminal and the server apparatus, and that manages predetermined medication history information,
the medication history management apparatus comprising a control section that performs, when a request for identification-numerical-value information is received from the first terminal, control so as to generate the identification-numerical-value information, to store the identification-numerical-value information in a predetermined storage section, and to display the identification-numerical-value information on the first terminal, wherein
the control section performs control so as to determine, when the identification-numerical-value information inputted from the second terminal, and patient-identification information and company-identification information stored in the second terminal are received from the second terminal, whether or not the identification-numerical-value information received from the second terminal is stored in the predetermined storage section,
to transmit, when the identification-numerical-value information received from the second terminal is stored in the predetermined storage section and access inquiring about whether or not the company-identification information is received in advance from the server apparatus, the patient-identification information received from the second terminal to the server apparatus, and
to generate, when redirect destination information that enables access to the server apparatus is received from the server apparatus that has received the patient-identification information, redirect setting information including the patient-identification information received from the second terminal and the redirect destination information, and to transmit the redirect setting information to the first terminal, wherein
the redirect setting information is information for the first terminal to request the server apparatus to display medication history information managed by the server apparatus on the first terminal.

10. A medication history management program to be executed by a computer that transmits and receives information to and from a first terminal and a second terminal and that manages a medication history, the program causing the computer to execute processing comprising:
generating identification-numerical-value information based on a request for the identification-numerical-value information received from the first terminal;
storing the identification-numerical-value information in a predetermined storage section and displaying the identification-numerical-value information on the first terminal;
receiving the identification-numerical-value information inputted from the second terminal and patient-identification information stored in the second terminal from the second terminal; and
reading, when the identification-numerical-value information received from the second terminal is stored in the predetermined storage section, medication history information corresponding to the patient-identification information from the predetermined storage section based on a request for the medication history information received from the first terminal and displaying the medication history information on the first terminal.

11. A medication history management program to be executed by a computer that transmits and receives information to and from a first terminal communicable with a server apparatus configured to manage predetermined medication history information, and a second terminal, and that manages predetermined medication history information, the program causing the computer to execute processing comprising:
generating the identification-numerical-value information based on a request for the identification-numerical-value information received from the first terminal;
storing the identification-numerical-value information in a predetermined storage section and displaying the identification-numerical-value information on the first terminal;
determining, when the identification-numerical-value information inputted from the second terminal, and patient-identification information and company-identification information stored in the second terminal are received from the second terminal, whether or not the identification-numerical-value information received from the second terminal is stored in the predetermined storage section;
identifying, when the identification-numerical-value information received from the second terminal is stored in the predetermined storage section, redirect destination information that enables access to the server apparatus based on the company-identification information received from the second terminal;
generating redirect setting information including the patient-identification information received from the second terminal and the redirect destination information;
and
transmitting the redirect setting information to the first terminal, wherein
the redirect setting information is information for the first terminal to request the server apparatus to display medication history information managed by the server apparatus on the first terminal.

12. A medication history management program to be executed by a computer that transmits and receives information to and from a first terminal communicable with a server apparatus configured to manage predetermined medication history information, a second terminal, and the server apparatus, and that manages predetermined medication history information, the program causing the computer to execute processing comprising:
generating identification-numerical-value information based on a request for the identification-numerical-value information received from the first terminal;
storing the identification-numerical-value information in a predetermined storage section and displaying the identification-numerical-value information on the first terminal;
determining, when the identification-numerical-value information inputted from the second terminal, and patient-identification information and company-identification information stored in the second terminal are received from the second terminal, whether or not the identification-numerical-value information received from the second terminal is stored in the predetermined storage section;
transmitting, when the identification-numerical-value information received from the second terminal is stored in the predetermined storage section and access inquiring about whether or not the company-identification information is received has been received in advance from the server apparatus, the patient-identification information received from the second terminal to the server apparatus;
generating, when redirect destination information that enables access to the server apparatus is received from the server apparatus that has received the patient-identification information, redirect setting information including the patient-identification information received from the second terminal and the redirect destination information; and
transmitting the redirect setting information to the first terminal, wherein
the redirect setting information is information for the first terminal to request the server apparatus to display medication history information managed by the server apparatus on the first terminal.
